# EUROPEAN PATENT APPLICATION

(11) **EP 4 534 695 A1**
(43) Date of publication of application: **09.04.2025**
(21) Application number: 23816320.8
(22) Date of filing: 26.05.2023
(51) Int. Cl.: C12Q 1/6883, G01N 33/68, G16B 40/20, G16B 40/00

(54) **GENETIC MARKERS FOR DIAGNOSIS OR PROGNOSIS PREDICTION OF DEGENERATIVE TEMPOROMANDIBULAR JOINT OSTEOARTHRITIS AND USE THEREOF**

(30) Priority: 30.05.2022 KR 20220065803; 09.12.2022 KR 20220171887
(71) Applicant: The Asan Foundation, Seoul 05505 (KR); UNIVERSITY OF ULSAN FOUNDATION FOR INDUSTRY COOPERATION, Nam-gu Ulsan 44610 (KR); Korea University Research and Business Foundation, Seoul 02841 (KR)
(72) Inventor: KIM, Yoon-Ji, Hanam-si Gyeonggi-do 13011 (KR); BAIK, Un-Bong, Seoul 06288 (KR); KIM, Wook, Seoul 01780 (KR); LEE, Kwang-Sig, Seoul 01695 (KR); JHA, Nayansi, Gyeonggi-do 16944 (KR)
(74) Representative: Dehns
(86) International application number: PCT/KR2023/007321
(87) International publication number: WO 2023/234659

(57) **Abstract**

The present invention relates to genetic markers for the diagnosis or prognosis prediction of degenerative temporomandibular joint osteoarthritis and use thereof, and more particularly use of a mutation of a gene selected from the group consisting of SMARCA2, NOTCH1 and a combination thereof; or a mutant protein encoded by a mutant gene produced by the mutation, for the diagnosis or prognosis prediction of temporomandibular joint osteoarthritis, and a method therefor. The markers provided by the present invention are closely related to degenerative temporomandibular joint osteoarthritis, and thus exhibit a very significant effect on the diagnosis or prognosis prediction of degenerative temporomandibular joint osteoarthritis. In addition, it is characterized by being capable of diagnosing degenerative temporomandibular joint osteoarthritis or predicting the prognosis thereof through a biological sample, such as saliva, which can be obtained by a non-invasive method. Moreover, unlike existing cases of simply diagnosing a disease with CT images or/and clinical information (EMR), a random forest-based deep & wide model that is constructed by a machine learning (deep learning) method taken together with the genetic mutation marker information of the present invention has excellent diagnostic and predictive abilities with an accuracy of 97%.

## Description

### [Technical Field]

The present invention relates to genetic markers for the diagnosis or prognosis prediction of degenerative temporomandibular joint osteoarthritis and use thereof, and more particularly use of a mutation of a gene selected from the group consisting of SMARCA2, NOTCH1, and a combination thereof; or a mutant protein, encoded by a mutant gene produced by the mutation, for the diagnosis or prognosis prediction of temporomandibular joint osteoarthritis, and a method therefor.

This application claims priority to Korean Patent Application No. 10-2022-0065803, filed on May 30, 2022, and Korean Patent Application No. 10-2022-0171887, filed on December 09, 2022, in the Korean Intellectual Property Office, and all the contents disclosed in the specification and drawings of the application are incorporated in this application.

### [Background Art]

Degenerative temporomandibular joint osteoarthritis (TMJ osteoarthritis, TMJ OA) is one of the osteoarthritis occurring in the temporomandibular joint (TMJ) and is intractable. This degenerative change can be caused by excessive use of the temporomandibular joint or systemic diseases, and is also known as a type of non-inflammatory disease rather than a true inflammatory response of the joint. TMJ osteoarthritis is a functional disorder that causes changes in the joint structure due to the destruction of the articular surface and underlying bone tissue, loss of articular cartilage, and degeneration, which can cause changes in the occlusion, leading to or worsening of symptoms such as open bite, so it should be considered during orthodontic diagnosis and treatment. The most common cause of osteoarthritis is an overload on the joint tissue. In the temporomandibular joint, catabolic and anabolic reactions occur in balance and function by adapting to the load applied to the joint. However, when the temporomandibular joint area becomes pathological, the balance between the two reactions is broken, tissues lower than the original tissues are synthesized, and adaptation to the load is not properly performed, resulting in degenerative changes in the temporomandibular joint. When degenerative changes occur in the temporomandibular joint, the surrounding structures change pathologically, or compensation for the joint changes occurs, resulting in various symptoms such as limited joint movement, joint noise, condyle entanglement, crepitus, and pain in the joint area. When the overload on the temporomandibular joint is reduced or the bone morphology remains but osteoarthritis is adapted, it is called TMJ osteoarthrosis.

In this way, TMJ degeneration destroys cartilage and changes the bone shape of the mandibular condyle and articular fossa. These morphological changes cause chronic pain and a decline in quality of life. Therefore, diagnosis of temporomandibular joint osteoarthritis (TMJ OA) before severe degeneration of cartilage and subchondral bone occurs may help prevent chronic pain and disability. However, since symptoms often do not appear before severe degeneration of the joint, it is difficult to diagnose osteoarthritis (OA) changes in the TMJ. In addition, diagnosis of temporomandibular joint osteoarthritis has mainly relied on imaging methods to date. This is likely to depend on the subjectivity of a reader and has limitations in determining whether the disease is currently progressive or not. In addition, previous studies have limitations in that they analyzed the concentration of bone metabolites in the synovial fluid of the temporomandibular joint or directly injected a probe into the joint tissue area and observed. The process of collecting synovial fluid is very invasive and there is a high possibility that the articular surface of the temporomandibular joint will be damaged during the collection process, and if a probe is directly inserted into the joint tissue area, there is a possibility of an inappropriate immune response against the probe itself.

### [Disclosure]

### [Technical Problem]

The present inventors have conducted research into a more reliable method for the diagnosis or prognosis prediction of degenerative temporomandibular joint osteoarthritis using a non-invasive method, and have confirmed that the diagnosis or prognosis prediction of degenerative temporomandibular joint osteoarthritis is possible through saliva when using genetic mutation markers provided by the present invention, and that this method shows a very significant effect in the diagnosis or prognosis prediction of the disease. Further, as a result of using machine learning (deep learning) to create a random forest-based Deep & Wide model on CT images and clinical information (EMR), i.e., the comprehensive information (data) of "CT images - genetic mutation - clinical information," along with the genetic marker information, the degenerative temporomandibular joint osteoarthritis diagnosis or prognosis prediction accuracy by the model was confirmed to be very high at 97%, thus completing the present invention.

Therefore, it is an object of the present invention to provide a composition for the diagnosis or prognosis prediction of degenerative temporomandibular joint osteoarthritis, comprising a preparation for detecting:
a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof; or
a mutant protein encoded by a mutant gene produced by the mutation.

It is another object of the present invention to provide a kit for the diagnosis or prognosis prediction of degenerative temporomandibular joint osteoarthritis, comprising the composition for the diagnosis or prognosis prediction of degenerative temporomandibular joint osteoarthritis of the present invention.

It is still another object of the present invention to provide a method for providing information necessary for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof, from the obtained nucleic acid sample or protein sample.

It is yet another object of the present invention to provide a method for providing information necessary for the diagnosis or prognosis prediction of degenerative temporomandibular joint osteoarthritis using random forest performed by a device for the diagnosis or prognosis estimation of degenerative temporomandibular joint osteoarthritis, the method comprising:
(a) obtaining information of (i) to (iii) below; and
   (i) information on a mutation of a gene selected from the group consisting of SMARCA2, NOTCH1, and a combination thereof in a biological sample isolated from a subject,
   (ii) information on a temporomandibular joint condyle morphology obtained by receiving a photographed image of a subject's temporomandibular joint and image-preprocessing the image, and
   (iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

It will be understood that technical problems of the present invention are not limited to the aforementioned problems and other technical problems not referred to herein will be clearly understood by those skilled in the art from disclosures below.

### [Technical Solution]

To achieve the purpose of the present invention, the present invention provides a composition for the diagnosis or prognosis prediction of degenerative temporomandibular joint osteoarthritis, comprising a preparation for detecting:
a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof; or
a mutant protein encoded by a mutant gene produced by the mutation.

In an embodiment of the present invention, detecting a mutation of the SMARCA2 gene may be, without being limited to,:
detecting a SMARCA2 mutant gene comprising a mutation in one or more single nucleotide polymorphism (SNP) sites selected from a group consisting of:
GenBank SNP database ID rs113070757, and
a 451st base site based on a base sequence of SEQ ID NO: 1 in a polynucleotide comprising the base sequence of SEQ ID NO: 1.

In a specific embodiment of the present invention, detecting a mutation of the SMARCA2 gene may be, without being limited to,:
detecting a SMARCA2 mutant gene comprising a mutation in one or more SNP sites selected from a group consisting of:
a deletion or duplication mutation of GenBank SNP database ID rs113070757; and
a mutation in which a 451st base is T based on the base sequence of SEQ ID NO: 1 in a polynucleotide comprising the base sequence of SEQ ID NO: 1.

In a specific embodiment of the present invention, the SMARCA2 mutant gene may encode, without being limited to, a SMARCA2 mutant protein comprising one or more mutations selected from the group consisting of a Ser151Cys substitution, a deletion of a 238th Gln, and a duplication of a 238th Gln based on SEQ ID NO: 2 in a SMARCA2 protein comprising an amino acid sequence of SEQ ID NO: 2.

In another embodiment of the present invention, detecting a mutation of the NOTCH1 gene may be, without being limited to,:
detecting a NOTCH1 mutant gene comprising a mutation in one or more SNP sites selected from a group consisting of:
GenBank SNP database IDs rs371414501, rs765380569, rs1589053221, rs763217096, and rs371742334.

In a specific embodiment of the present invention, detecting a mutation of the NOTCH1 gene may be, without being limited to,:
detecting a NOTCH1 mutant gene comprising a mutation in one or more SNP sites selected from a group consisting of:
a mutation in which a base of GenBank SNP database ID rs371414501 is T;
a mutation in which a base of GenBank SNP database ID rs765380569 is G;
a mutation in which a base of GenBank SNP database ID rs1589053221 is T;
a mutation in which a base of GenBank SNP database ID rs763217096 is T; and
a mutation in which a base of GenBank SNP database ID rs371742334 is A.

In a specific embodiment of the present invention, the NOTCH1 mutant gene may encode, without being limited to, a NOTCH1 mutant protein comprising one or more amino acid substitutions selected from a group consisting of Ala1944Thr, Glu1563Asp, Met2196Ile, Asp909Asn, and Arg2159Cys based on SEQ ID NO: 4 in a NOTCH1 protein comprising an amino acid sequence represented by SEQ ID NO: 4.

In an embodiment of the present invention, in the composition for the diagnosis or prognosis prediction of degenerative temporomandibular joint osteoarthritis of the present invention, the preparation for detecting a mutation of the gene; or a mutant protein encoded by a mutant gene produced by the mutation may comprise, without being limited to, a primer, a probe or an antibody.

To achieve the other purpose of the present invention, the present invention provides a kit for the diagnosis or prognosis prediction of degenerative temporomandibular joint osteoarthritis, comprising the composition for the diagnosis or prognosis prediction of degenerative temporomandibular joint osteoarthritis of the present invention.

In an embodiment of the present invention, the kit of the present invention may comprise, without being limited to, an instruction manual that teaches contents comprising the method for providing information necessary for the diagnosis or prognosis prediction of degenerative temporomandibular joint osteoarthritis of the present invention.

To achieve another purpose of the present invention, the present invention provides a method for providing information necessary for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof from the obtained nucleic acid sample or protein sample.

To achieve another purpose of the present invention, the present invention provides a method for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof from the obtained nucleic acid sample or protein sample.

In an embodiment of the present invention, the method for providing information necessary for the diagnosis or prognosis prediction of degenerative temporomandibular joint osteoarthritis of the present invention may further comprise, without being limited to, the following step (c):
(c) determining that degenerative temporomandibular joint osteoarthritis has occurred when the confirmed genotype or protein type is a mutant.

In an embodiment of the present invention, the biological sample used in the method for providing information necessary for the diagnosis or prognosis prediction of degenerative temporomandibular joint osteoarthritis of the present invention may be one or more selected from a group consisting of saliva, blood, oral epithelial cells, temporomandibular joint tissue (cells), and temporomandibular joint synovial fluid, but the present invention is not limited thereto.

In an embodiment of the present invention, the genotype confirmation of the step (b) in the method for providing information necessary for the diagnosis or prognosis prediction of degenerative temporomandibular joint osteoarthritis of the present invention may be performed by one method selected from the group consisting of sequencing, exome sequencing, next generation sequencing (NGS), pyrosequencing, microarray hybridization, allele-specific PCR, dynamic allele-specific hybridization, PCR extension analysis, PCR-SSCP method, Taqman technique and a combination of two or more thereof, but the present invention is not limited thereto.

In another embodiment of the present invention, the genotype or protein type confirmation of the step (b) in the method for providing information necessary for the diagnosis or prognosis prediction of degenerative temporomandibular joint osteoarthritis of the present invention may be performed by:
one method selected from a group consisting of microarray analysis, polymerase chain reaction (PCR), hybridization with allele-specific probes, enzyme-assisted mutation detection, ligation chain reaction (LCR), oligonucleotide ligation assay (OLA), flow cytometric heterozygosity analysis, chemical cleavage of mismatches, mass spectrometry, RNA sequencing, single strand conformational polymorphism (SSCP), denaturing gradient gel electrophoresis (DGGE), temperature gradient gel electrophoresis (TGGE), restriction fragment polymorphism, sequential analysis of gene expression (SAGE), reverse transcription polymerase chain reaction (RT-PCR), competitive RT-PCR, real-time RT-PCR, quantitative or semi-quantitative RT-PCR, quantitative or semi-quantitative real-time RT-PCR, in situ hybridization, fluorescence in situ hybridization (FISH), RNase protection assay (RPA), northern blotting, southern blotting, RNA sequencing, DNA chip, RNA chip, immunoassay, western blotting, enzyme linked immunoassay (ELISA), radioimmunoassay, radioimmunodiffusion, Ouchterlony immunodiffusion, rocket immunoelectrophoresis, immunohistochemical staining, immunoprecipitation assay, complement fixation assay, flow cytometry (FACS), protein chip and a combination of two or more thereof, but the present invention is not limited thereto.

In an embodiment of the present invention, the method for providing information necessary for the diagnosis or prognosis prediction of degenerative temporomandibular joint osteoarthritis of the present invention may be applied to Asians, but the present invention is not limited thereto.

In a specific embodiment of the present invention, the Asians may be Koreans, but the present invention is not limited thereto.

To achieve another purpose of the present invention, the present invention provides a method for providing information necessary for the diagnosis or prognosis prediction of degenerative temporomandibular joint osteoarthritis using random forest performed by a device for the diagnosis or prognosis estimation the prognosis of degenerative temporomandibular joint osteoarthritis, the method comprising:
(a) obtaining information of (i) to (iii) below; and
   (i) information on a mutation of a gene selected from the group consisting of SMARCA2, NOTCH1, and a combination thereof in a biological sample isolated from a subject,
   (ii) information on a temporomandibular joint condyle morphology obtained by receiving a photographed image of a subject's temporomandibular joint and image-preprocessing the image, and
   (iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

To achieve another purpose of the present invention, the present invention provides a method for the diagnosis or prognosis prediction of degenerative temporomandibular joint osteoarthritis using random forest performed by the device for the diagnosis or prognosis estimation of degenerative temporomandibular joint osteoarthritis, the method comprising:
(a) obtaining information of (i) to (iii) below; and
   (i) information on a mutation of a gene selected from the group consisting of SMARCA2, NOTCH1, and a combination thereof in a biological sample isolated from a subject,
   (ii) information on a temporomandibular joint condyle morphology obtained by receiving a photographed image of a subject's temporomandibular joint and image-preprocessing the image, and
   (iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

In an embodiment of the present invention, the image may be obtained through a lateral cephalogram, a computed tomography (CT), a transcranial radiograph, a panoramic radiograph, or a magnetic resonance imaging (MRI) scan, but the present invention is not limited thereto.

In an embodiment of the present invention, the information on the temporomandibular joint condyle morphology may be obtained by a method comprising, without being limited to,:
extracting a target region comprising a temporomandibular joint condyle region from a computed tomography (CT);
extracting feature points of the condyle from the extracted target region; and
estimating outline information on the condyle based on the extracted cervical vertebrae feature points.

In an embodiment of the present invention, the random forest-based degenerative temporomandibular joint osteoarthritis diagnosis or prognosis estimation model may be learned through information analyzed based on, without being limited to,:
plural photographed temporomandibular joint images for which a diagnosis or prognosis of temporomandibular joint osteoarthritis has been determined,
plural clinical information for which a diagnosis or prognosis of temporomandibular joint osteoarthritis has been determined, and
genetic mutation information for which a diagnosis or prognosis of temporomandibular joint osteoarthritis has been determined.

In an embodiment of the present invention, the photographed temporomandibular joint images may be analyzed with a single shot detector (SSD) to produce four classifications below and confidence scores therefor, without being limited to,:
an osteophyte type; a micro-condyle type; a post-flattening type; or an ant/sup flattening type.

To achieve another purpose of the present invention, the present invention provides a use of a preparation for detecting a mutation of a gene selected from a group consisting of SMARCA2 (SWI/SNF Related, Matrix Associated, Actin Dependent Regulator Of Chromatin, Subfamily A, Member 2), NOTCH1 (Notch Receptor 1) and a combination thereof, or a mutant protein encoded by a mutant gene produced by the mutation; or composition comprising the preparation for the diagnosis or prognosis prediction of degenerative temporomandibular joint osteoarthritis.

To achieve another purpose of the present invention, the present invention provides a use of a preparation for detecting a mutation of a gene selected from a group consisting of SMARCA2 (SWI/SNF Related, Matrix Associated, Actin Dependent Regulator Of Chromatin, Subfamily A, Member 2), NOTCH1 (Notch Receptor 1) and a combination thereof, or a mutant protein encoded by a mutant gene produced by the mutation; or composition comprising the preparation for manufacturing a preparation for the diagnosis or prognosis prediction of degenerative temporomandibular joint osteoarthritis.

### [Advantageous effects]

Genetic mutation markers (comprising mutant protein markers encoded by mutant genes produced by mutations) provided by the present invention are closely related to degenerative temporomandibular joint osteoarthritis, and thus exhibit a very significant effect on the diagnosis or prognosis prediction of degenerative temporomandibular joint osteoarthritis. In addition, it is characterized by being capable of the diagnosis or prognosis prediction of degenerative temporomandibular joint osteoarthritis or predicting the prognosis thereof through a biological sample, such as saliva, which can be obtained by a non-invasive method. Moreover, unlike existing cases of simply diagnosing a disease with CT images or/and clinical information (EMR), a random forest-based deep & wide model that is constructed by a machine learning (deep learning) method combined with an existing method, the genetic mutation marker information of the present invention, CT images and clinical information (EMR) has excellent diagnostic and predictive abilities with an accuracy of 97%.

### [Description of Drawings]

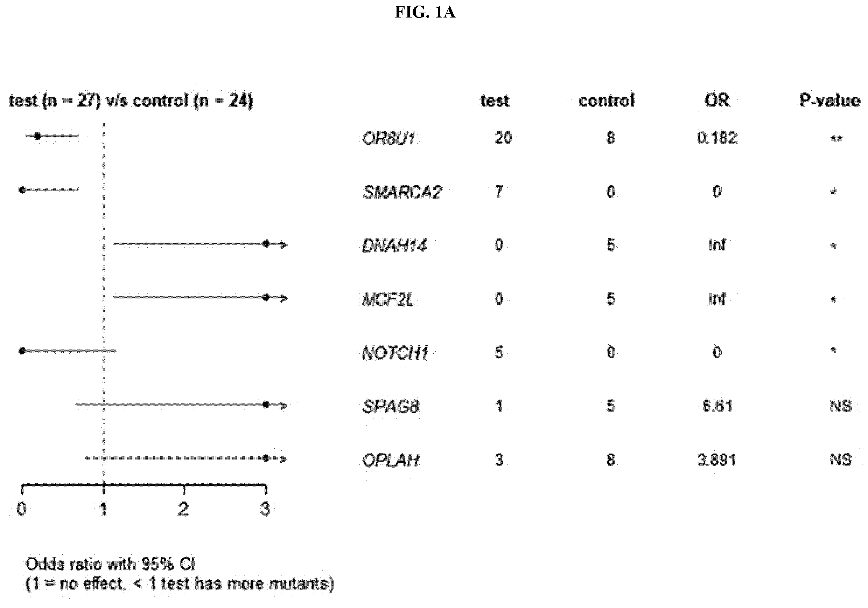

FIG. 1A graphically illustrates the results of mutation tendency analysis of 7 genes, OR8U1, SMARCA2, DNAH14, MCF2L, NOTCH1, SPAG8, and OPLAH, that showed significantly higher mutation rates in patients with temporomandibular joint osteoarthritis or a control group (left), and illustrates the results matched with the contents of Table 1 (right).

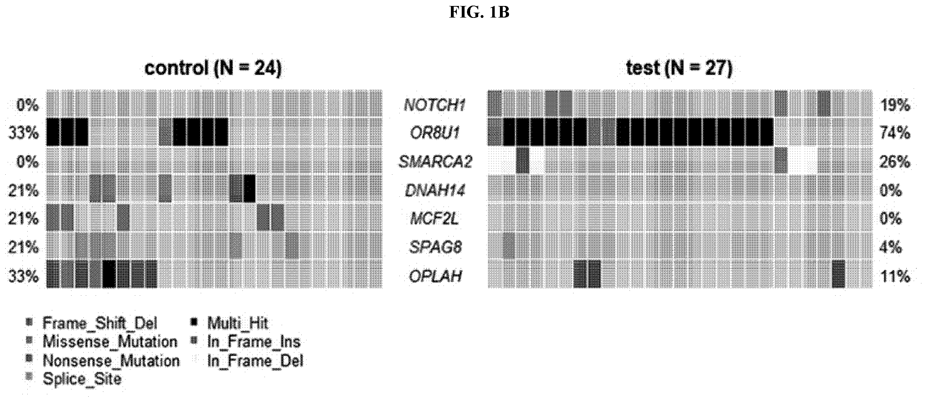

FIG. 1B illustrates specific mutation types for the seven genes, OR8U1, SMARCA2, DNAH14, MCF2L, NOTCH1, SPAG8, and OPLAH, and the number of subjects in which each of the mutations is found (1 cell corresponds to 1 person).

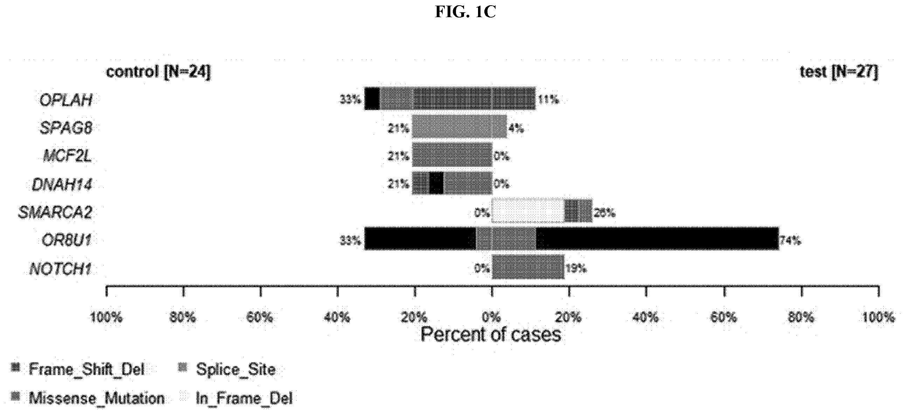

FIG. 1C graphically illustrates the results of FIG. 1B converted into probabilities, and represents the specific mutation types discovered for the seven genes, OR8U1, SMARCA2, DNAH14, MCF2L, NOTCH1, SPAG8, and OPLAH, and the probability of each mutation.

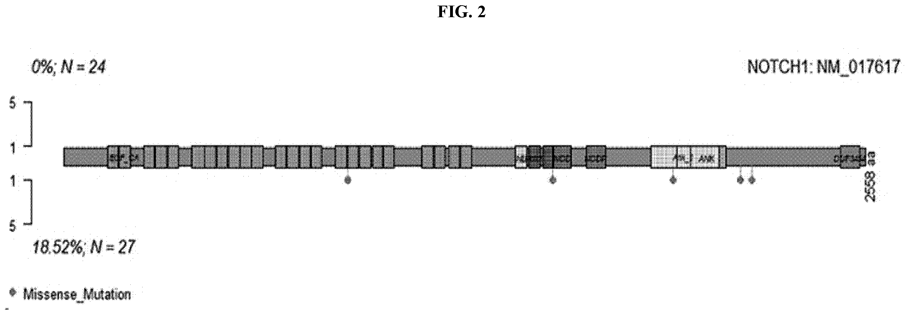

FIG. 2 graphically illustrates the location of each NOTCH1 SNP missense mutation of the present invention, which is a specific marker for the diagnosis of degenerative temporomandibular joint osteoarthritis (see Table 2).

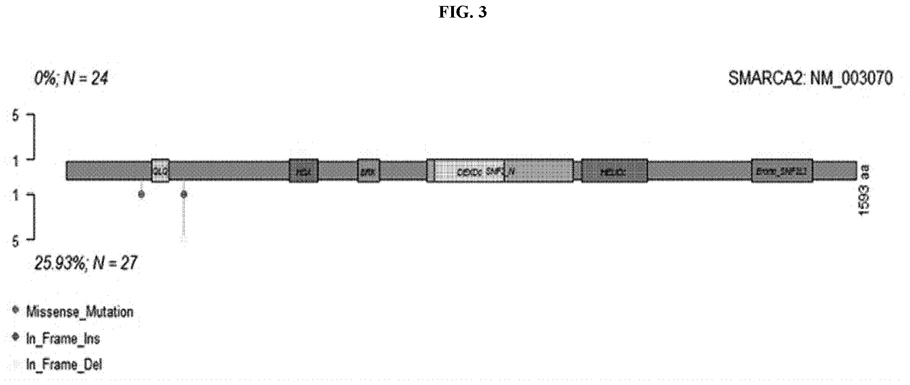

FIG. 3 graphically illustrates the locations of each SMARCA2 SNP missense mutation, inframe insertion, and inframe deletion of the present invention, which are markers specific to the diagnosis of degenerative temporomandibular joint osteoarthritis (see Table 3).

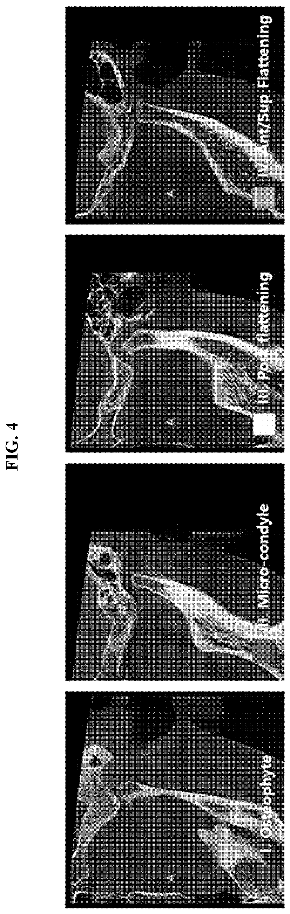

FIG. 4 illustrates the criteria for the temporomandibular joint CT image class classification corresponding to the variable list v002 in Table 4 in a specific diagnosis and prognosis prediction model of the present invention for degenerative temporomandibular joint osteoarthritis, and particularly illustrates that it is classified into four criteria according to the shape of the temporomandibular joint CT mandibular condyle: Class I. osteophyte, Class II. micro-condyle, Class III. posterior flattening, and Class IV. anterior/superior flattening.

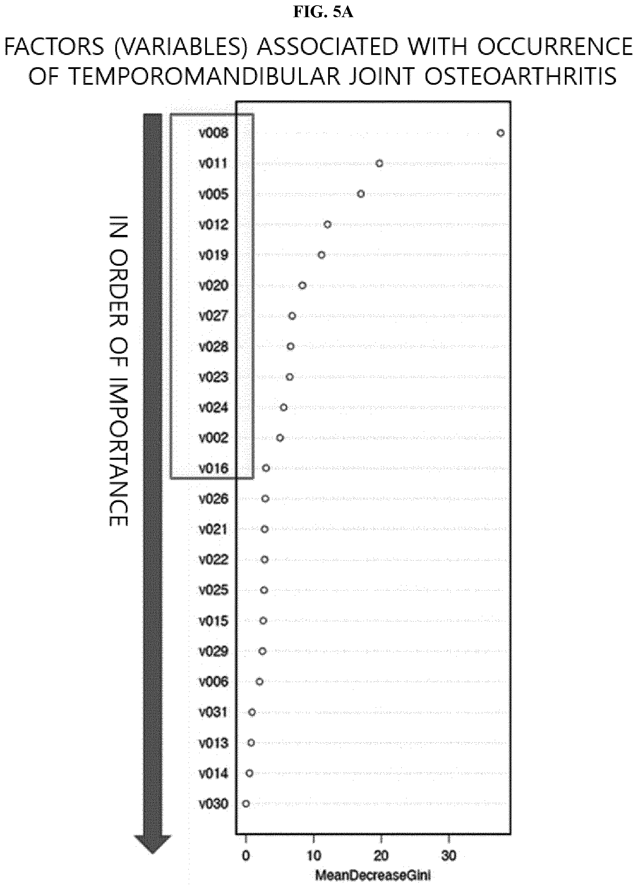

FIG. 5A is a random forest plot illustrating the importance ranking of variables in predicting temporomandibular joint osteoarthritis among the variables in Table 4 in the degenerative temporomandibular joint osteoarthritis diagnosis and prognosis prediction model of the present invention, and the top 12 important variables among them.

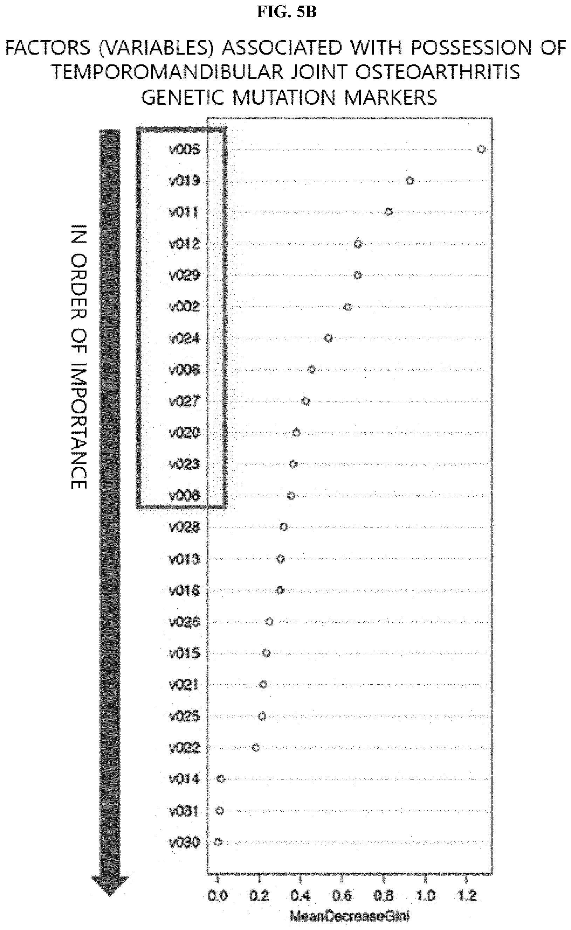

FIG. 5B is a random forest plot illustrating the importance ranking of variables in predicting the presence of Korean-specific genetic mutations (SMARCA2, Notch1) listed in Tables 2 and 3 among the variables in Table 4 in the degenerative temporomandibular joint osteoarthritis diagnosis and prognosis prediction model of the present invention, and the top 12 important variables among them.

### [Best Mode]

### Definition

Unless otherwise defined, all technical and scientific terms used herein have the same meaning as commonly understood by those skilled in the art.

In the present invention, the term "degenerative temporomandibular joint osteoarthritis" refers to intractable osteoarthritis that occurs in the temporomandibular joint (TMJ), commonly called the temporomandibular joint. The most common cause of this disease is excessive load on the joint tissue, which occurs when the articular surface is damaged by articular disc displacement and retrodiscitis. In addition, the disease is a type of non-inflammatory disease in which the joint surface and underlying bone tissue are destroyed, and often causes pain when bone changes actively occur. As commonly used in the art, the terms "temporomandibular joint osteoarthritis", "TMJ osteoarthritis", "TMJ OA", etc. may be used interchangeably in this specification. According to American Academy of Orofacial Pain, TMJ osteoarthritis is classified into primary and secondary. The primary TMJ osteoarthritis is characterized by the absence of distinct local or systemic factors. However, the secondary TMJ osteoarthritis means that it is related to a previous traumatic event or disease. In the present invention, the type of the degenerative temporomandibular joint osteoarthritis is not specifically limited.

In this specification, "polynucleotide" or "nucleic acid" means deoxyribonucleotide (DNA) or ribonucleotide (RNA) in single- or double-stranded form. Known analogs of natural nucleotides that hybridize to nucleic acids in a manner similar to naturally occurring nucleotides are also included. In general, DNA is composed of four bases: adenine (A), guanine (G), cytosine (C), and thymine (T), while RNA has uracil (U) instead of thymine. In the double strand of nucleic acid, A forms hydrogen bonds with T or U, and C forms hydrogen bonds with G. This relationship between bases is called "complementary." In addition, in this specification, "genomic DNA (gDNA)" means the total base sequences of genes of an individual, which means DNA containing almost complete genetic information.

In this specification, when indicating a mutation of a gene, "c." means a mutation in a protein-encoding region, and "(base position) (base letter) (symbol >) (base letter)" means that the previously indicated base at the corresponding base position is replaced with the subsequently indicated base.

In this specification, "protein" is used interchangeably with "polypeptide" or "peptide", and, for example, refers to a polymer of amino acid residues, such as those commonly found in proteins in their natural state.

The one letter (three letters) of each of the amino acids used in this specification means each of the following amino acids according to the standard abbreviation convention in the field of biochemistry: A (Ala): Alanine; C (Cys): Cysteine; D (Asp): Aspartic acid; E (Glu): Glutamic acid; F (Phe): Phenylalanine; G (Gly): Glycine; H (His): Histidine; I (IIe): Isoleucine; K (Lys): Lysine; L (Leu): Leucine; M (Met): Methionine; N (Asn): Asparagine; O (Ply): Pyrrolysine; P (Pro): Proline; Q (Gln): Glutamine; R (Arg): Arginine; S (Ser): Serine; T (Thr): Threonine; U (Sec): Selenocysteine; V (Val): Valine; W (Trp): Tryptophan; Y (Tyr): Tyrosine.

The "(one letter (three letters) for amino acid) (amino acid position) (one letter (three letters) for amino acid)" indicated in this specification means that, at the corresponding amino acid position of the wild-type protein, the amino acid indicated in advance is replaced with the amino acid indicated in the subsequent amino acid.

In this specification, "diagnosis" includes determining the susceptibility of an object to the occurrence of a corresponding (target) disease, determining whether an object currently has a corresponding (target) disease, determining the prognosis of an object suffering from a corresponding (target) disease (e.g., identifying a degenerative temporomandibular joint osteoarthritis condition, determining the stage of degenerative temporomandibular joint osteoarthritis, or determining the responsiveness of degenerative temporomandibular joint osteoarthritis to treatment), or monitoring the condition of an object to provide information about the efficacy of treatment of a corresponding (target) disease.

In this specification, the term "prognosis" includes the possibility of progression of a corresponding (target) disease, particularly, the prediction in terms of the degree of disease remission, disease regeneration, and recurrence. Preferably, the prognosis in the present invention may mean the possibility that the disease of a patient with degenerative temporomandibular joint osteoarthritis will be cured.

SMARCA2 (SWI/SNF Related, Matrix Associated, Actin Dependent Regulator Of Chromatin, Subfamily A, Member 2) in the present invention is preferably derived from a human, and the sequence information of mRNA and protein of the human SMARCA2 gene is known in the GenBank (NCBI) database under accession numbers of NM_003070 and NP_003061, respectively.

In the present invention, NOTCH1 (Notch Receptor 1) is preferably derived from a human, and the sequence information of mRNA and protein of the human NOTCH1 gene is known in the GenBank (NCBI) database under accession numbers of NM_017617 and NP_060087, respectively.

In this specification, the term "polymorphism" means the occurrence of two or more alternative sequences or alleles within a genetically determined population, and the term "single nucleotide polymorphism (SNP)" means polymorphism of one base. Specifically, it refers to the diversity of a single base (A, T, C or G) occurring between members of a species or between pairs of chromosomes of an individual in a genome. For example, if there are differences in a single base as in three DNA fragments (e.g., AAGT[A/A]AG, AAGT[A/G]AG, AAGT[G/G]AG) from different individuals, the different bases are called two alleles (A or G), and generally, almost all SNPs have two alleles. In addition, when a SNP is genetically closely associated with a specific disease, the SNP also means that a mutation has occurred in one base at a specific location, compared to a confirmed normal or wild-type (WT) individual or allele.

A mutation in SMARCA2 or NOTCH1 gene (particularly, SNP site), which is a maker for degenerative temporomandibular joint osteoarthritis diagnosis according to the present invention, is located in a protein-encoding region (coding region) of SMARCA2 or NOTCH1, and a genetic mutation trait (particularly, SNP allele) related to degenerative temporomandibular joint osteoarthritis may cause damage to a SMARCA2 or NOTCH1 protein function.

Hereinafter, the present invention is described in detail.

The present invention provides a composition for the diagnosis or prognosis prediction of degenerative temporomandibular joint osteoarthritis, comprising a preparation for detecting:
a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof; or
a mutant protein encoded by a mutant gene produced by the mutation.

In a preferred aspect of the present invention, detecting a mutation of the SMARCA2 gene may be detecting a SMARCA2 mutant gene comprising a mutation in one or more SNP sites selected from the following groups, which correspond to markers provided by the present invention:
GenBank SNP database ID rs113070757, and
a 451st base site based on the base sequence of SEQ ID NO: 1 in a polynucleotide comprising the base sequence of SEQ ID NO: 1.

In a more preferred embodiment of the aspect, detecting a mutation of the SMARCA2 gene may be detecting a SMARCA2 mutant gene comprising a mutation in one or more SNP sites selected from the following groups, which correspond to markers provided by the present invention:
a deletion or duplication mutation of GenBank SNP database ID rs113070757; and
a mutation in which a 451st base is T based on the base sequence of SEQ ID NO: 1 in a polynucleotide comprising the base sequence of SEQ ID NO: 1.

In the present invention, the deletion or duplication mutation of the GenBank SNP database ID rs113070757 may be a deletion or duplication mutation of 705th to 707th bases based on the base sequence of SEQ ID NO: 1 in a polynucleotide comprising the base sequence of SEQ ID NO: 1, but the present invention is not limited thereto.

In addition, the SMARCA2 mutant gene according to the aspect may encode a SMARCA2 mutant protein comprising one or more mutations selected from the group consisting of a Ser151Cys substitution, a deletion of the 238th Gln, and a duplication of the 238th Gln, based on the SEQ ID NO: 2, in the SMARCA2 protein comprising the amino acid sequence of SEQ ID NO: 2, and the mutant proteins correspond to markers provided by the present invention.

In a preferred aspect of the present invention, detecting a mutation of the NOTCH1 gene may be detecting a NOTCH1 mutant gene comprising a mutation in one or more SNP sites selected from the following groups, which correspond to markers provided by the present invention:
a group consisting of GenBank SNP database IDs rs371414501, rs765380569, rs1589053221, rs763217096, and rs371742334.

In a more preferred embodiment of the aspect, detecting a mutation of the NOTCH1 gene may be detecting a NOTCH1 mutant gene comprising a mutation in one or more SNP sites selected from the following groups, which correspond to markers provided by the present invention:
a mutation in which the base of GenBank SNP database ID rs371414501 is T;
a mutation in which the base of GenBank SNP database ID rs765380569 is G;
a mutation in which the base of GenBank SNP database ID rs1589053221 is T;
a mutation in which the base of GenBank SNP database ID rs763217096 is T; and
a mutation in which the base of GenBank SNP database ID rs371742334 is A.

In the present invention, the mutation in which the base of GenBank SNP database ID rs371414501 is T may be a mutation in which the 5830th base is A based on the base sequence of SEQ ID NO: 3 in a polynucleotide comprising the base sequence of SEQ ID NO: 3, but the present invention is not limited thereto.

In the present invention, the mutation in which the base of the GenBank SNP database ID rs765380569 is G may be a mutation in which the 4689th base is C based on the base sequence of SEQ ID NO: 3 in the polynucleotide comprising the base sequence of SEQ ID NO: 3, but the present invention is not limited thereto.

In the present invention, the mutation in which the base of the GenBank SNP database ID rs1589053221 is T may be a mutation in which the 6588th base is A based on the base sequence of SEQ ID NO: 3 in the polynucleotide comprising the base sequence of SEQ ID NO: 3, but the present invention is not limited thereto.

In the present invention, the mutation in which the base of the GenBank SNP database ID rs763217096 is T may be a mutation in which the 2725th base is A based on the base sequence of SEQ ID NO: 3 in the polynucleotide comprising the base sequence of SEQ ID NO: 3, but the present invention is not limited thereto.

In the present invention, the mutation in which the base of the GenBank SNP database ID rs371742334 is A may be a mutation in which the 6475th base is T based on the base sequence of SEQ ID NO: 3 in the polynucleotide comprising the base sequence of SEQ ID NO: 3, but the present invention is not limited thereto.

In addition, in the aspect, the NOTCH1 mutant gene may encode a NOTCH1 mutant protein comprising one or more amino acid substitutions selected from a group consisting of Ala1944Thr, Glu1563Asp, Met2196Ile, Asp909Asn, and Arg2159Cys based on SEQ ID NO: 4 in the NOTCH1 protein comprising the amino acid sequence represented by SEQ ID NO: 4, and the mutant protein corresponds to a marker provided by the present invention.

In an aspect of the present invention, in the composition for the diagnosis or prognosis prediction of degenerative temporomandibular joint osteoarthritis of the present invention, a preparation for detecting a mutation of the gene; or a mutant protein encoded by a mutant gene produced by the mutation may comprise a primer, a probe or an antibody, but the present invention is not limited thereto.

Specifically, the gene mutation functioning as a marker in the present invention (comprising mRNA of the mutant gene due to the mutation) may be detected using a primer (primer pair) or probe that specifically binds to a corresponding gene including the mutation.

In addition, a mutant protein encoded by the mutant gene that is produced by the gene mutation functioning as a marker in the present invention may be detected using an antibody (an antibody that specifically binds to the mutant protein) specific to the mutant protein.

In addition, the present invention provides a use of a preparation for detecting a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1) and a combination thereof, or a mutant protein encoded by a mutant gene produced by the mutation; or a composition comprising the preparation for the diagnosis or prognosis prediction of degenerative temporomandibular joint osteoarthritis.

In addition, the present invention provides a use of a preparation for detecting a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1) and a combination thereof, or a mutant protein encoded by a mutant gene produced by the mutation; or a composition comprising the preparation for manufacturing a preparation for the diagnosis or prognosis prediction of degenerative temporomandibular joint osteoarthritis.

In addition, the present invention provides a kit for the diagnosis or prognosis prediction of degenerative temporomandibular joint osteoarthritis, comprising the composition for the diagnosis or prognosis prediction of degenerative temporomandibular joint osteoarthritis of the present invention.

The type of the kit is not particularly limited so long as it is a kit used for a genotype confirmation method (gene analysis method) or/and protein-type confirmation (protein analysis method) known in the art described below. For example, the kit may be a microarray kit, a gene amplification kit, or an immunoassay kit, but the present invention is not limited thereto.

In addition, the kit of the present invention may comprise, separately from the preparation for detecting the markers of the present invention described above, one or more other compositions suitable for the analysis method, the label described above, and a material and device used for detecting the same.

In an aspect of the present invention, the kit of the present invention may comprise an instruction manual that teaches contents comprising the method for providing information necessary for the diagnosis or prognosis prediction of degenerative temporomandibular joint osteoarthritis of the present invention, which is described below, but the present invention is not limited thereto.

In addition, the present invention provides a method for providing information necessary for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof from the obtained nucleic acid sample or protein sample.

In addition, the present invention provides a method for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof from the obtained nucleic acid sample or protein sample.

In an aspect of the present invention, the biological sample may mean any biological sample that can be genetically tested, comprising, but not limited to, saliva, blood, oral epithelial cells, temporomandibular joint tissue (cells), and temporomandibular joint synovial fluid.

In the step (a), a method for extracting (or isolating) a nucleic acid (a polynucleotide such as DNA or RNA (particularly, mRNA)) or a protein from a biological sample is well-known in the art and may be used without limitation in the present invention.

The specific type of genotypes or protein types of one selected from the group consisting of SMARCA2, NOTCH1 and a combination thereof, as confirmed in the step (b), will be understood by referring to the marker types (particularly, SNP) of the present invention which have been specifically described above with regard to the composition for diagnosing or predicting the prognosis of degenerative temporomandibular joint osteoarthritis of the present invention.

In a preferred aspect of the present invention, the genotype confirmation of the step (b) may be performed by one method selected from the group consisting of sequencing, exome sequencing, next generation sequencing (NGS), pyrosequencing, microarray hybridization, allele-specific PCR, dynamic allele-specific hybridization, PCR extension analysis, PCR-SSCP method, Taqman technique and a combination of two or more thereof, but the present invention is not limited thereto.

For example, Whole-exome sequencing (WES) is a technique that analyzes approximately 3×10⁷ bp of base sequence that encodes all proteins, known as exome, in the genome. Although the exome accounts for less than 2% of the human genome, mutations in this region can have much more serious consequences than mutations that occur in the remaining 98%. The purpose of WES is to find genetic mutations such as disease-causing mutations inherited in a Mendelian manner or single nucleotide polymorphisms (SNPs), which increase the risk of developing common and complex diseases, through a filtering process in big data containing all protein-coding regions.

In another embodiment of the present invention, the genotype or protein-type confirmation of the step (b) in the method of providing information necessary for the diagnosis or prognosis prediction of degenerative temporomandibular joint osteoarthritis of the present invention may be performed by:
one method selected from the group consisting of microarray analysis, polymerase chain reaction (PCR), hybridization with allele-specific probes, enzyme-assisted mutation detection, ligation chain reaction (LCR), oligonucleotide ligation assay (OLA), flow cytometric heterozygosity analysis, chemical cleavage of mismatches, mass spectrometry, RNA sequencing, single strand conformational polymorphism (SSCP), denaturing gradient gel electrophoresis (DGGE), temperature gradient gel electrophoresis (TGGE), restriction fragment polymorphism, sequential analysis of gene expression (SAGE), reverse transcription polymerase chain reaction (RT-PCR), competitive RT-PCR, real-time RT-PCR, quantitative or semi-quantitative RT-PCR, quantitative or semi-quantitative real-time RT-PCR, in situ hybridization, fluorescence in situ hybridization (FISH), RNase protection assay (RPA), northern blotting, southern blotting, RNA sequencing, DNA chip, RNA chip, immunoassay, western blotting, enzyme linked immunoassay (ELISA), radioimmunoassay, radioimmunodiffusion, Ouchterlony immunodiffusion, rocket immunoelectrophoresis, immunohistochemical staining, immunoprecipitation assay, complement fixation assay, flow cytometry (FACS), protein chip and a combination of two or more thereof, but the present invention is not limited thereto.

In a preferred aspect of the present invention, the method for providing information necessary for the diagnosis or prognosis prediction of degenerative temporomandibular joint osteoarthritis of the present invention may further comprise the following step (c):
(c) determining that degenerative temporomandibular joint osteoarthritis has occurred when the confirmed genotype or protein type is a mutant.

In a preferred aspect of the present invention, the method for providing information necessary for the diagnosis or prognosis prediction of degenerative temporomandibular joint osteoarthritis of the present invention may be applied to Asians, but the present invention is not limited thereto. In a more preferred aspect of the present invention, the Asians may be Koreans, but the present invention is not limited thereto.

In addition, the present invention provides a method for providing information necessary for the diagnosis or prognosis prediction of degenerative temporomandibular joint osteoarthritis using random forest performed by a device for the diagnosis or prognosis estimation the prognosis of degenerative temporomandibular joint osteoarthritis, the method comprising:
(a) obtaining information of (i) to (iii) below; and
   (i) information on a mutation of a gene selected from the group consisting of SMARCA2, NOTCH1, and a combination thereof in a biological sample isolated from a subject,
   (ii) information on a temporomandibular joint condyle morphology obtained by receiving a photographed image of a subject's temporomandibular joint and image-preprocessing the image, and
   (iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

In addition, the present invention provides a method for the diagnosis or prognosis prediction of degenerative temporomandibular joint osteoarthritis using random forest performed by the device for the diagnosis or prognosis estimation of degenerative temporomandibular joint osteoarthritis, the method comprising:
(a) obtaining information of (i) to (iii) below; and
   (i) information on a mutation of a gene selected from the group consisting of SMARCA2, NOTCH1, and a combination thereof in a biological sample isolated from a subject,
   (ii) information on a temporomandibular joint condyle morphology obtained by receiving a photographed image of a subject's temporomandibular joint and image-preprocessing the image, and
   (iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

In an aspect of the present invention, the image may be obtained through a lateral cephalogram, a computed tomography (CT), a transcranial radiograph, a panoramic radiograph, or a magnetic resonance imaging (MRI) scan, but the present invention is not limited thereto.

In an aspect of the present invention, the CT scan may be any type of CT, for example, cone beam computed tomography (CBCT) and conventional CT, but the present invention is not limited thereto.

In an aspect of the present invention, the information on the temporomandibular joint condyle morphology may be obtained by a method comprising:
extracting a target region comprising a temporomandibular joint condyle region from a computed tomography (CT);
extracting feature points of the condyle from the extracted target region; and
estimating outline information on the condyle based on the extracted cervical vertebrae feature points, but the present invention is not limited thereto.

In an aspect of the present invention, the random forest-based degenerative temporomandibular joint osteoarthritis diagnosis or prognosis estimation model may be learned through information analyzed based on the following information:
plural photographed temporomandibular joint images for which a diagnosis or prognosis of temporomandibular joint osteoarthritis has been determined,
plural clinical information for which a diagnosis or prognosis of temporomandibular joint osteoarthritis has been determined, and
genetic mutation information for which a diagnosis or prognosis of temporomandibular joint osteoarthritis has been determined, but the present invention is not limited thereto.

In an aspect of the present invention, the photographed temporomandibular joint images may be analyzed with a single shot detector (SSD) to produce the following four classifications and confidence scores therefor, but the present invention is not limited thereto:
an osteophyte type; a micro-condyle type; a post-flattening type; or an ant/sup flattening type.

In an aspect of the present invention, the SSD and the Random Forest are responsible for the "deep" and "wide" parts of the Deep & Wide model, in that the results analyzed by the SSD, which is a "deep learning" model, become input data for the Random Forest, which is a "wide learning" model.

In addition, the present invention provides a method for providing information for determining/analyzing whether a subject is highly susceptible to developing degenerative temporomandibular joint osteoarthritis, the method characterized by identifying a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof in isolated biological samples from a subject suspected of having degenerative temporomandibular joint osteoarthritis and a control group, and then deeming that the subject is at a high risk of developing degenerative temporomandibular joint osteoarthritis when a mutation occurs in the genotype or the protein type.

In addition, the present invention provides a method for treating degenerative temporomandibular joint osteoarthritis, the method comprising:
(a) obtaining a nucleic acid or protein sample from a biological sample isolated from a subject;
(b) identifying a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof in the obtained nucleic acid sample or protein sample;
(c) determining that the subject has degenerative temporomandibular joint osteoarthritis when a mutation occurs in the genotype or protein type (i.e., determining that degenerative temporomandibular joint osteoarthritis has developed); and
(d) treating the degenerative temporomandibular joint osteoarthritis of the subject when the subject is determined as having degenerative temporomandibular joint osteoarthritis.

In the present invention, the treating step may comprise drug treatment (administrating a medicine for degenerative temporomandibular joint osteoarthritis), physical treatment, and the like, but the present invention is not limited thereto.

In addition, the present invention provides a method for characterizing a responder to a degenerative temporomandibular joint osteoarthritis medicine for treating degenerative temporomandibular joint osteoarthritis, the method comprising:
(S1) identifying a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof in a biological sample isolated from a subject; and
(S2) determining as a responder to degenerative temporomandibular joint osteoarthritis medicine when a mutation occurs in the genotype or protein type measured in the step (S1).

The degenerative temporomandibular joint osteoarthritis medicine may comprise antiinflammatory analgesic drugs, muscle relaxants, etc. used in the art to treat degenerative temporomandibular joint osteoarthritis, but there is no limitation on the type thereof.

Now, the present invention will be described in more detail with reference to the following preferred examples. These examples are provided for illustrative purposes only and should not be construed as limiting the scope and spirit of the present invention.

### Example 1. Discovery of novel biomarkers for diagnosis of degenerative temporomandibular joint osteoarthritis

### 1-1. Saliva-based whole exome sequencing

From each of 27 Korean patients with degenerative temporomandibular joint osteoarthritis (experimental group), 2 ml of saliva was collected, and whole exome sequencing (Macrogen) was performed to create a list of genetic mutations. In addition, as a control group, a total of 2ml of saliva was sampled from 24 healthy Koreans without temporomandibular joint disease, and whole exome sequencing was performed to perform genetic analysis.

As a result of the experiment, it was confirmed that various types of mutations were found for 40 or more different genes.

### 1-2. Discovery of degenerative temporomandibular joint osteoarthritis diagnosis-specific marker genes and mutations

Among the numerous mutations found in Example 1-1, genes useful for diagnosing degenerative temporomandibular joint osteoarthritis were primarily selected. The specific method was as follows:
The base sequences obtained from the experimental group and control group samples through whole exome sequencing were aligned to the Genome Reference Consortium Human Build 37 [hg19], and then mutations (variants) were detected using gatk (version 3.4.0), a genome analysis toolkit from the Broad Institute, and saved in variant call format (VCF). The VCF was annotated with variant annotation using Variant Effect Prediction (VEP, version 104.3) of the European Bioinformatics Institute (ENSEMBL) and saved in the mutation annotation format (MAF). After reading MAF with mutation analysis tool Maftools (Mayakonda et al., 2018, Genome Res.), rare mutations with DP (depth) of 30 or more and allele frequency of 0.001 or less based on mutation databases (1000 Genomes and gnomAD) for normal individuals were selected. For all the mutations selected in this way, the Fisher test was performed using Maftools, and genes (differentially mutated genes) showing statistically significant differences (p-value<0.10) between the experimental group and the control group were detected. Results are shown in Table 1 below.

**[Table 1]**

| | **test** | **control** | **OR** | **P-value** |
|---|---|---|---|---|
| *OR8U1* | 20 | 8 | 0.182 | ** |
| *SMARCA2* | 7 | 0 | 0 | * |
| *DNAH14* | 0 | 5 | Inf | * |
| *MCF2L* | 0 | 5 | Inf | * |
| *NOTCH1* | 5 | 0 | 0 | * |
| *SPAG8* | 1 | 5 | 6.61 | NS |
| *OPLAH* | 3 | 8 | 3.891 | NS |

As a result, as shown in Table 1 and FIG. 1A, seven genes, OR8U1, SMARCA2, DNAH14, MCF2L, NOTCH1, SPAG8, and OPLAH, which showed significantly higher mutations in the experimental group or the control group compared to each other, were selected.

Among these, to discover specific markers for the diagnosis of degenerative temporomandibular joint osteoarthritis, the diagnostic utility of the genes was further analyzed. As a result, as shown in FIG. 1A, mutations in the SMARCA2 and NOTCH1 genes were finally found only in patients with temporomandibular joint osteoarthritis, and were not found in the control group composed of normal people.

Specifically, as shown in FIG. 1B, the specific genetic mutations that showed significant differences were examined. In the case of the SMARCA2 gene, inframe deletion occurred in 4 out of 7 patients, inframe insertion occurred in 1 patient, and missense mutation occurred in 1 patient. In the case of the NOTCH1 gene, missense mutations were confirmed in all 5 patients.

In addition, as shown in FIG. 1C, when examining the rates of genetic mutation occurrence in the temporomandibular joint osteoarthritis patient group and the control group, SMARCA2 mutation occurred in 7 out of 27 (26%), and the NOTCH1 mutation occurred in 5 out of 27 (19%).

Mutations appearing in the two genes SMARCA2 and NOTCH1 were confirmed to be genetic mutations associated with temporomandibular joint osteoarthritis, and among them, mutations described in Tables 2 and 3 were specifically selected as markers specific to the diagnosis of degenerative temporomandibular joint osteoarthritis.

Table 2 shows detailed information including the NOTCH1 gene mutation (SNP) location information and protein mutation information generated accordingly, which function as a specific marker for the diagnosis of degenerative temporomandibular joint osteoarthritis of the present invention.

In addition, Table 3 shows detailed information including the SMARCA2 gene mutation (SNP) location information and the protein mutation information generated accordingly, which function as a specific marker for the diagnosis of degenerative temporomandibular joint osteoarthritis of the present invention.

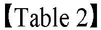

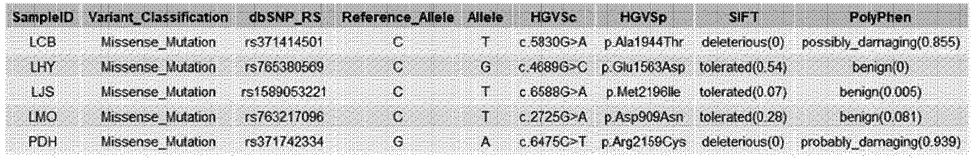

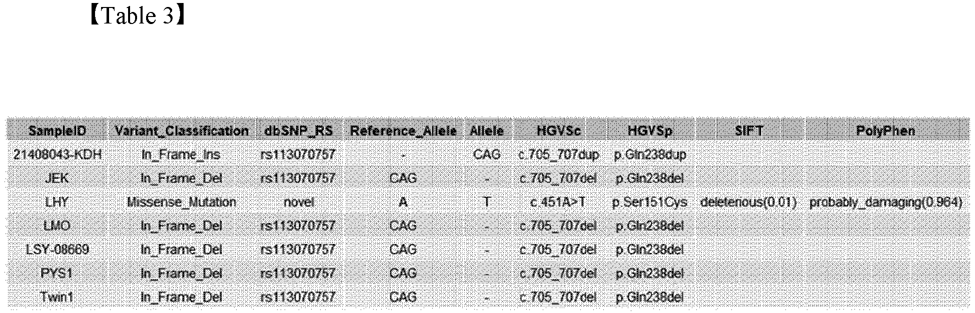

As can be confirmed in Table 2, five missense mutations having NOTCH1 gene mutation (SNP) were newly identified as markers specific to degenerative temporomandibular joint osteoarthritis diagnosis. Specifically, It was confirmed that the mutation where GenBank SNP database ID rs371414501 base was substituted with T, the mutation where rs765380569 base was substituted with G, the mutation where rs1589053221 base was substituted with T, the mutation where rs763217096 base was substituted with T, and the mutation where rs371742334 base was substituted with A had excellent discriminatory power as degenerative temporomandibular joint osteoarthritis diagnosis markers. FIG. 2 is a diagram simply illustrating the location of each of the NOTCH1 gene mutation (SNP) missense mutations described in Table 2.

In addition, as confirmed in Table 3, one Inframe deletion, one Inframe insertion, and one Missense mutation were newly identified as SMARCA2 gene mutation (SNP)-specific markers for degenerative temporomandibular joint osteoarthritis diagnosis. It was confirmed that mutations, in which the CAG base (705th to 707th bases based on SEQ ID NO: 1) in GenBank SNP database ID rs113070757 was deleted or duplicated and the 451st base based on the SMARCA2 sequence of SEQ ID NO: 1 was substituted with T, were excellently discriminatory as markers for the diagnosis of degenerative temporomandibular joint osteoarthritis. FIG. 3 is a diagram simply illustrating the location of each of the SMARCA2 SNP missense mutation, inframe insertion, and Inframe deletion described in Table 3.

### Example 2. Completion of Deep & Wide model using marker gene mutations specific to diagnosis of degenerative temporomandibular joint osteoarthritis

### 2-1. Application of random forest-based Deep and Wide model to comprehensive marker gene mutation, CT image, and EMR (clinical information) information of the present invention specific to diagnosis of degenerative temporomandibular joint osteoarthritis

Big data-based risk factor information among the genetic mutation information, CT images, and EMR (clinical information) shown in Tables 2 and 3 newly identified in the present invention were merged as variables (Table 4) to develop a unique diagnosis and prognosis prediction model of the present invention in the form of a Decision Tree and Random Forest-based Deep and Wide model.

The results analyzed by the Single Shot Detector (SSD), a "deep learning" model, become the input data for the random forest, a "wide Learning" model. In this respect, SSD and the random forest are responsible for the "deep" and "wide" parts of the Deep & Wide model.

Table 4 below shows the list of variables in the unique diagnosis and prognosis prediction model of the present invention, which is constructed in the form of the deep and wide mode.

**[Table 4]**

| Variable name | Description |
|---|---|
| v032 | Condyle shape abnormality 0: No 1: Yes / **FIG. 5A** **dependent variable** |
| v004 | SNP (SMARCA2, Notch1) 0: No 1: Yes / **FIG. 5B** **dependent variable** |
| v002 | Confidence (class distinction and probability value, between 0 and 1, for the corresponding class by the pre-built AI algorithm in the temporomandibular joint CBCT image)_**see** **FIG. 4** |
| v005 | Age |
| v006 | Sex (m:1/f:2) |
| v008 | Diagnosis (k07.65 (1)/k07.66 (2)/ k07.60 (3)/k07.63 (4)) Temporomandibular joint disorder subclassification |
| | [K07.60] Temporomandibular joint internal disease |
| | [K07.61] Temporomandibular joint noise |
| | [K07.62] Recurrent dislocation and subluxation of temporomandibular joint |
| | [K07.63] Pain of temporomandibular joint, not elsewhere classified |
| | [K07.64] Stiffness of the temporomandibular joint, not elsewhere classified |
| | [K07.65] Degenerative joint disease of temporomandibular joint |
| | [K07.66] Disorder of masticatory muscles |
| | [K07.68] Other specified temporomandibular joint disorders |
| | [K07.69] Unspecified temporomandibular joint disorder |
| v011 | Overbite Degree of coverage of upper and lower front teeth |
| v012 | Overjet Anteroposterior distance between upper and lower front teeth |
| v013 | Muscle pain Right ( (-):0 / (masseter pain):1 / (temporalis pain):2 / (sternocleidomastoid pain):3) |
| v014 | Muscle pain Left ( (-):0 / (masseter pain):1 / (temporalis pain):2 / (sternocleidomastoid pain):3) |
| v015 | Pain on palpation of temporomandibular joint capsule ( (-):0 / (+):1) RIGHT |
| v016 | Pain on palpation of temporomandibular joint capsule ( (-):0 / (+):1) LEFT |
| v019 | Joint noise (none (0)/crepitus (1)/poping (2)/clicking (3)) RIGHT |
| v020 | Joint noise (none (0)/crepitus (1)/poping (2)/clicking (3)) LEFT |
| v021 | **Joint pain** ( (-):0/ (+):1) RIGHT |
| v022 | **Joint pain** ( (-):0/ (+):1) LEFT |
| v023 | Molar relationship (classI (1) / classII (2)/classIII (3) RIGHT |
| v024 | Molar relationship (classI (1) / classII (2) / classIII (3) LEFT |
| v025 | Number of missing teeth Maxilla |
| v026 | Number of missing teeth Mandibular |
| v027 | Total number of prostheses: UPPER |
| v028 | Total number of prostheses: LOWER |
| v029 | Orthodontic treatment (Not received (0)/received extraction orthodontic treatment (1)/received non-extraction orthodontic treatment (2) |
| v030 | Rheumatoid arthritis ( (-):0/ (+):1)) |
| v031 | Oral malformation habit (none (0)/teeth clenching (1)/teeth bruxism (2)/teeth clenching+teeth bruxism (3)) |

The temporomandibular joint CT image class classification corresponding to the variable list v002 in Table 4 was classified into four criteria according to the shape of the mandibular condyle in the temporomandibular joint CT: Class I. osteophyte, Class II. micro-condyle, Class III. posterior flattening, and Class IV. anterior/superior flattening, as shown in FIG. 4. Single Shot Detector (SSD) performs a classification task to predict which category a specific object or lesion belongs to, and also calculates a confidence score that indicates how certain the classification result is. The confidence score can be considered as a statistic summarizing the characteristics of image data, the confidence score was used as input data of the random forest along with EMR data and the gene mutation (marker) information of the present invention, and a Deep & Wide Model was constructed to synthesize these various data and models.

In the model of the present invention, the importance rankings of the top 12 variables predicting temporomandibular joint osteoarthritis according to the recent results on Random Forest Variable Importance are as follows (see FIG. 5A):
v008 (Diagnosis 1 K07.65 / 2 K07.66 / 3 K07.60 / 4 K07.63) [37],
v011 (OB) [20],
v005 (Age) [17],
v012 (OJ) [12],
v019 (Noise Right) [11],
v020 (Noise Left) [8],
v027 (Number of Upper Prostheses) [7],
v028 (Number of Lower Prostheses) [7],
v023 (Molar Relationship Right) [7],
v024 (Molar Relationship Left) [6],
v002 (Single Shot Detector Confidence Score-Image-based diagnostic prediction value)[5],
v016 (Capsule Palpation Left) [3]

In the model of the present invention, according to the recent results on Random Forest Variable Importance, the importance rankings of the top 12 variables predicting whether Koreans have unique genetic mutations (SMARCA2, Notch1) listed in Tables 2 and 3 are as follows (see FIG. 5B):
v005 (Age) [1.3],
v019 (Noise Right) [0.9],
v011 (OB) [0.8],
v012 (OJ) [0.7],
v029 (Brace Wearing) [0.7],
v002 (Single Shot Detector Confidence Score) [0.6] (Image-based diagnosis prediction value),
v024 (Molar Relationship Left) [0.5],
v006 (Sex) [0.5],
v027 (Number of Upper Prostheses) [0.4],
v020 (Noise Left) [0.4],
v023 (Molar Relationship Right) [0.4],
v008 (Diagnosis 1 K07.65 / 2 K07.66 / 3 K07.60 / 4 K07.63) [0.4]

### 2-2. Verification of unique temporomandibular joint osteoarthritis diagnosis and prognosis prediction technology of present invention

The accuracy of the degenerative temporomandibular joint osteoarthritis diagnosis and prognosis prediction model (method) of the present invention constructed in Example 2-1 was verified through external validation. The process of dividing the training set and test set and predicting using the random forest was repeated 50 times while changing the number of seeds, and then the average of the predictive abilities was calculated to maximize external validation.

As a result, the degenerative temporomandibular joint osteoarthritis diagnosis and prognosis prediction model (method) of the present invention was confirmed to have excellent diagnosis or predictive abilities with an accuracy of 97%. This result is in contrast to the previous result "an accuracy of 77% in diagnosing degenerative temporomandibular joint osteoarthritis" obtained by applying a single shot detector to image data consisting of 1,700 "no temporomandibular joint osteoarthritis" and 1,814 "with temporomandibular joint osteoarthritis" categories according to the existing international standard Diagnostic Criteria for Temporomandibular Disorders (DC/TMD, Schiffman et al. 2014).

The aforementioned description of the present invention is provided by way of example and those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention.

### [Industrial Applicability]

Genetic mutation markers provided by the present invention are closely related to degenerative temporomandibular joint osteoarthritis, and the diagnosis or prognosis prediction of degenerative temporomandibular joint osteoarthritis is possible through a biological sample that can be obtained by a non-invasive method such as saliva, so the present invention is industrially applicable.

## Claims

1. A composition for the diagnosis or prognosis prediction of degenerative temporomandibular joint osteoarthritis, comprising a preparation for detecting:
a mutation of a gene selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof; or
a mutant protein encoded by a mutant gene produced by the mutation.

2. The composition of claim 1, wherein detecting a mutation of the SMARCA2 gene is:
detecting a SMARCA2 mutant gene comprising a mutation in one or more single nucleotide polymorphism (SNP) sites selected from a group consisting of:
GenBank SNP database ID rs113070757, and
a 451st base site based on a base sequence of SEQ ID NO: 1 in a polynucleotide comprising the base sequence of SEQ ID NO: 1.

3. The composition of claim 2, wherein detecting a mutation of the SMARCA2 gene is:
detecting a SMARCA2 mutant gene comprising a mutation in one or more SNP sites selected from a group consisting of:
a deletion or duplication mutation of GenBank SNP database ID rs113070757; and
a mutation in which a 451st base is T based on the base sequence of SEQ ID NO: 1 in a polynucleotide comprising the base sequence of SEQ ID NO: 1.

4. The composition of claim 2 or 3, wherein the SMARCA2 mutant gene encodes a SMARCA2 mutant protein comprising one or more mutations selected from the group consisting of a Ser151Cys substitution, a deletion of a 238th Gln, and a duplication of a 238th Gln based on SEQ ID NO: 2 in a SMARCA2 protein comprising an amino acid sequence of SEQ ID NO: 2.

5. The composition of claim 1, wherein detecting a mutation of the NOTCH1 gene is:
detecting a NOTCH1 mutant gene comprising a mutation in one or more SNP sites selected from a group consisting of:
GenBank SNP database IDs rs371414501, rs765380569, rs1589053221, rs763217096, and rs371742334.

6. The composition of claim 5, wherein detecting a mutation of the NOTCH1 gene is:
detecting a NOTCH1 mutant gene comprising a mutation in one or more SNP sites selected from a group consisting of:
a mutation in which a base of GenBank SNP database ID rs371414501 is T;
a mutation in which a base of GenBank SNP database ID rs765380569 is G;
a mutation in which a base of GenBank SNP database ID rs1589053221 is T;
a mutation in which a base of GenBank SNP database ID rs763217096 is T; and
a mutation in which a base of GenBank SNP database ID rs371742334 is A.

7. The composition of claim 5 or 6, wherein the NOTCH1 mutant gene encodes a NOTCH1 mutant protein comprising one or more amino acid substitutions selected from a group consisting of Ala1944Thr, Glu1563Asp, Met2196Ile, Asp909Asn, and Arg2159Cys based on SEQ ID NO: 4 in a NOTCH1 protein comprising an amino acid sequence represented by SEQ ID NO: 4.

8. The composition of claim 1, wherein the preparation comprises a primer, a probe, or an antibody.

9. A kit for the diagnosis or prognosis prediction of degenerative temporomandibular joint osteoarthritis, comprising the composition according to claim 1.

10. A method for providing information necessary for the diagnosis or prognosis prediction of degenerative temporomandibular joint osteoarthritis, the method comprising:
(a) obtaining a nucleic acid or protein sample from an isolated biological sample; and
(b) confirming a genotype or protein type of one selected from the group consisting of SMARCA2 (SWI/SNF Related, Matrix Associated, Actin Dependent Regulator Of Chromatin, Subfamily A, Member 2), NOTCH1 (Notch Receptor 1), and a combination thereof from the obtained nucleic acid sample or protein sample.

11. The method of claim 10, further comprising (c) determining that degenerative temporomandibular joint osteoarthritis has occurred when the confirmed genotype or protein type is a mutant.

12. The method of claim 10, wherein the biological sample is one or more selected from a group consisting of saliva, blood, oral epithelial cells, temporomandibular joint tissue (cells), and temporomandibular joint synovial fluid.

13. The method of claim 10, wherein genotype confirmation of the step (b) is performed by one method selected from the group consisting of sequencing, exome sequencing, next generation sequencing (NGS), pyrosequencing, microarray hybridization, allele-specific PCR, dynamic allele-specific hybridization, PCR extension analysis, PCR-SSCP method, Taqman technique and a combination of two or more thereof.

14. The method of claim 10, wherein the genotype or protein type confirmation of the step (b) is performed by one method selected from a group consisting of microarray analysis, polymerase chain reaction (PCR), hybridization with allele-specific probes, enzyme-assisted mutation detection, ligation chain reaction (LCR), oligonucleotide ligation assay (OLA), flow cytometric heterozygosity analysis, chemical cleavage of mismatches, mass spectrometry, RNA sequencing, single strand conformational polymorphism (SSCP), denaturing gradient gel electrophoresis (DGGE), temperature gradient gel electrophoresis (TGGE), restriction fragment polymorphism, sequential analysis of gene expression (SAGE), reverse transcription polymerase chain reaction (RT-PCR), competitive RT-PCR, real-time RT-PCR, quantitative or semi-quantitative RT-PCR, quantitative or semi-quantitative real-time RT-PCR, in situ hybridization, fluorescence in situ hybridization (FISH), RNase protection assay (RPA), northern blotting, southern blotting, RNA sequencing, DNA chip, RNA chip, immunoassay, western blotting, enzyme linked immunoassay (ELISA), radioimmunoassay, radioimmunodiffusion, Ouchterlony immunodiffusion, rocket immunoelectrophoresis, immunohistochemical staining, immunoprecipitation assay, complement fixation assay, flow cytometry (FACS), protein chip and a combination of two or more thereof.

15. The method of claim 10, wherein the method is applied to Asians.

16. The method of claim 15, wherein the Asians comprise Koreans.

17. The kit of claim 9, wherein the kit comprises an instruction manual that teaches contents comprising the method of claim 10.

18. A method for providing information necessary for the diagnosis or prognosis prediction of degenerative temporomandibular joint osteoarthritis using random forest performed by a device for the diagnosis or prognosis estimation the prognosis of degenerative temporomandibular joint osteoarthritis, the method comprising:
(a) obtaining information of (i) to (iii) below; and
(i) information on a mutation of a gene selected from the group consisting of SMARCA2, NOTCH1, and a combination thereof in a biological sample isolated from a subject,
(ii) information on a temporomandibular joint condyle morphology obtained by receiving a photographed image of a subject's temporomandibular joint and image-preprocessing the image, and
(iii) clinical information (EMR) of the subject,
(b) obtaining information on an occurrence or progression of degenerative temporomandibular joint osteoarthritis by using a random forest-based degenerative temporomandibular arthritis diagnosis or prognosis estimation model that receives the obtained genetic mutation information, temporomandibular joint condyle morphology information, and clinical information.

19. The method of claim 18, wherein the image is obtained through a lateral cephalogram, a computed tomography (CT), a transcranial radiograph, a panoramic radiograph, or a magnetic resonance imaging (MRI) scan.

20. The method of claim 18, wherein the information on the temporomandibular joint condyle morphology is obtained by a method comprising:
extracting a target region comprising a temporomandibular joint condyle region from a computed tomography (CT);
extracting feature points of the condyle from the extracted target region; and
estimating outline information on the condyle based on the extracted cervical vertebrae feature points.

21. The method of claim 18, wherein the random forest-based degenerative temporomandibular joint osteoarthritis diagnosis or prognosis estimation model is learned through information analyzed based on:
plural photographed temporomandibular joint images for which a diagnosis or prognosis of temporomandibular joint osteoarthritis has been determined,
plural clinical information for which a diagnosis or prognosis of temporomandibular joint osteoarthritis has been determined, and
genetic mutation information for which a diagnosis or prognosis of temporomandibular joint osteoarthritis has been determined.

22. The method of claim 18 or 21, wherein the photographed temporomandibular joint images are analyzed with a single shot detector (SSD) to produce four classifications below and confidence scores therefor:
an osteophyte type; a micro-condyle type; a post-flattening type; or an ant/sup flattening type.

23. Use of a preparation for detecting a mutation of a gene selected from a group consisting of SMARCA2 (SWI/SNF Related, Matrix Associated, Actin Dependent Regulator Of Chromatin, Subfamily A, Member 2), NOTCH1 (Notch Receptor 1), and a combination thereof; or a mutant protein encoded by a mutant gene produced by the mutation for the diagnosis or prognosis prediction of degenerative temporomandibular joint osteoarthritis.

24. Use of a preparation for detecting a mutation of a gene selected from a group consisting of SMARCA2 (SWI/SNF Related, Matrix Associated, Actin Dependent Regulator Of Chromatin, Subfamily A, Member 2), NOTCH1 (Notch Receptor 1), and a combination thereof; or a mutant protein encoded by a mutant gene produced by the mutation for manufacturing a preparation for the diagnosis or prognosis prediction of degenerative temporomandibular joint osteoarthritis.
